# EUROPÄISCHE PATENTANMELDUNG

(11) **EP 0 992 216 A1**
(43) Veröffentlichungstag der Anmeldung: **12.04.2000**
(21) Anmeldenummer: 99119746.8
(22) Anmeldetag: 06.10.1999
(51) Int. Cl.: A61B 5/042, A61B 5/00, A61B 7/02, A61B 8/12

(54) **Vitaldiagnose- und Überwachungsgerät**

(30) Priorität: 06.10.1998 DE 29817812 U
(71) Anmelder: Baumeier, Wolfgang, Dr.med., 23562 Lübeck (DE)
(72) Erfinder: Baumeier, Wolfgang, Dr. med., D-23562 Lübeck (DE)
(74) Vertreter: DIEHL GLAESER HILTL & PARTNER

(57) **Zusammenfassung**

Die Erfindung bezieht sich auf ein Vitaldiagnosegerät zur Erfassung wenigstens der Herzfunktion mit einer Sonde, die mit wenigstens zwei voneinander beabstandet am Umfang der Sonde angordneten Elektroden versehen ist, und einer Einrichtung zum Anzeigen der von der Sonde erfassten, einen Rückschluss auf Vitalfunktionen erlaubenden Signale. Durch eine flexible, ein Einführen in die Speiseröhre erlaubende Ausgestaltung ist die Sonde über ein mehradriges Kabel mit der gesondert ausgebildeten, außerhalb des Körpers des Patienten verbleibenden Auswerte- und Anzeigeeinrichtung verbunden.

## Beschreibung

Die Erfindung betrifft ein Vitaldiagnose- und Überwachungsgerät zur Überwachung wenigstens der Herzfunktionen, mit einer füllerartig ausgebildeten Sonde, die mit wenigstens zwei voneinander beabstandet am Umfang der Sonde angeordneten Elektroden versehen ist, und einer Einrichtung zum Anzeigen der von der Sonde erfassten, einen Rückschluss auf Vitalfunktionen erlaubenden Signale.

Aus der DE 36 36 996 C1 ist ein Herzdiagnosegerät bekannt, das füllerartig ausgebildet ist und an seinem Umfang mit wenigstens zwei Elektroden versehen ist. Dieses Herzdiagnosegerät dient dazu, im Notfall dem behandelnden Arzt eine Aussage über die Herzfunktion des Patienten zu erlauben.

Das Gerät ist insofern unzulänglich, als es für eine länger andauernde Überwachung ungeeignet ist.

Der Erfindung liegt die Aufgabe zu Grunde, ein Vitaldiagnosegerät der eingangs genannten Art zu schaffen, das eine Notfallüberwachung erlaubt.

Erfindungsgemäß wird diese Aufgabe gelöst durch eine ein Einführen in die Speiseröhre erlaubende Sonde zur Erfassung aller für eine Überwachung erforderlichen Funktionen, die über ein Kabel mit einer außerhalb des Patienten verbleibenden Auswerte- und Anzeigeeinrichtung verbunden ist.

Eine bevorzugte Ausführungsform eines Diagnosegerätes gemäß der Erfindung ist gekennzeichnet durch eine flexible, ein Einführen in die Speiseröhre erlaubende Ausgestaltung, wobei die Sonde über ein Kabel mit der gesondert ausgebildeten, außerhalb des Körpers des Patienten verbleibenden Auswerte- und Anzeigeeinrichtung verbunden ist, und die Sonde mit einem Temperatursensor, einem Sensor zur Erfassung der Sauerstoffsättigung, einem zur Erfassung der Herztöne und der Atemgeräusche dienenden Schallsensor und mit einem zur Erfassung der pulssynchronen Blutbewegungen dienenden Doppler-Schallkopf versehen ist, der seine Umgebung segmentweise über einen Raumwinkel von 360° abtastet und dass in der Auswerte- und Anzeigeeinrichtung dasjenige Raumsegment ausgewertet und angezeigt wird, das das kräftigste pulssynchrone Flüssigkeitsströmungssignal liefert.

Das erfindungsgemäß ausgebildete Vitaldiagnosegerät ist vor allem für den Notfalleinsatz innerhalb und außerhalb der Klinik gedacht. Der Notfallarzt (oder auch das medizinische Assistenzpersonal) führt die Sonde in die Speiseröhre ein und aktiviert diese über das mit der Sonde über ein dieses haltende und die elektrische (ggf. auch fluidische) Kommunikation herstellendes Kabel. Die Anzeigeeinrichtung ist dazu eingerichtet, die von der Sonde erfassten Vitalparameter in geeigneter Form anzuzeigen.

Der Vorteil des Gerätes besteht darin, dass es auch in Extremsituationen, wie großer Kälte, Feuchtigkeit, Dunkelheit, einer unruhigen Umgebung oder eines unruhigen Patienten sowie bei einer extrem schlechten Kreislaufsituation, bei der die üblichen Messverfahren versagen, die Erfassung von Vitalfunktionen erlaubt.

Bei dem bevorgzugten Diagnosegerät gemäß der Erfindung wird
die gesamte Zirkumferenz der Sonde durch Doppler-Signal abgetastet,
herzschlagsynchrone Blutbewegungen in der Umgebung der Speiseröhre erfasst, wobei das ausgeprägteste, gerichtete Strömungsprofil in dem Querschnitts-Segment zu erwarten sein wird, das der Aorta (Hauptschlagader) unmittelbar benachbart liegt.

Abhängig von der Tiefe, in die die Sonde in die Speiseröhre vorgeschoben wird, könnte auch die linke Herzkammer benachbart zu der Speiseröhre liegen, welche auch ein deutliches andersartiges Strömungsprofil liefern kann.

Es gilt, mittels des Schallkopfes oder der Schallköpfe das Segment auszuwerten, welches das kräftigste pulssynchrone Flüssigkeitsströmungssignal liefert. Dieses Signal soll qualitativ bewertet zur Anzeige gebracht werden, weil es eine Aussage über die Pumpfunktion des Herzens (keine, geringe, mäßige, kräftige, ... Pumpfunktion) liefert.

Diese Aussage hat unmittelbare Behandlungskonsequenzen für den kritsch Kranken und ggfs. reanimationspflichtigen Patienten. Erst durch Kombination mit EKG-Signal und Pulsoxmetrie über die gleiche Sonde lässt sich die Lebensbedrohung, die auch wiederum von der Kerntemperatur abhängig ist, ableiten.

Alle diese Messungen werden in der Speiseröhre noch verlässliche Signale liefern, wenn an der Körperoberfläche dies bereits nicht mehr möglich ist. Eine Auswertung des EKG-Signals kann dahingehend erfolgen, festzustellen, ob die Sonde in die korrekte Tiefe vorgeschoben ist.

Das angeschlossene mobile Signalverarbeitungs- und anzeigegerät wird die Signale bewerten und verknüpfen. Die Qualität der Vitalfunktionen des kritisch erkrankten Menschen lässt sich unmittelbar und bewertet hinsichtlich der Signalqualität ablesen,

## Patentansprüche

1. Vitaldiagnosegerät zur Erfassung wenigstens der Herzfunktion mit einer füllerartigen ausgebildeten Sonde, die mit wenigstens zwei voneinander beabstandet am Umfang der Sonde angordneten Elektroden versehen ist, und einer Einrichtung zum Anzeigen der von der Sonde erfassten, einen Rückschluss auf Vitalfunktionen erlaubenden Signale, gekennzeichnet durch eine flexible, ein Einführen in die Speiseröhre erlaubende Ausgestaltung, wobei die Sonde über ein mehradriges Kabel mit der gesondert ausgebildeten, außerhalb des Körpers des Patienten verbleibenden Auswerte- und Anzeigeeinrichtung verbunden ist.

2. Vitaldiagnosegerät nach Anspruch 1, dadurch gekennzeichnet, dass die Sonde weiter mit einem Temperatursensor versehen ist.

3. Vitaldiagnosegerät nach Anspruch 1 oder 2, dadurch gekennzeichnet, dass die Sonde weiter mit einem Sensor zur Erfassung der Sauerstoffsättigung versehen ist.

4. Vitaldiagnosegerät nach Anspruch 3, dadurch gekennzeichnet, dass die die Sauerstoffsättigung messende Sonde nach dem Prinzip der Reflexions-Pulsoxymetrie arbeitet.

5. Vitaldiagnosegerät nach mindestens einem der vorangehenden Ansprüche, dadurch gekennzeichnet, dass das Gerät weiter mit einem zur Erfassung der Herztöne und der Atemgeräusche dienenden Schallsensor versehen ist.

6. Vitaldiagnosegerät nach Anspruch 5, dadurch gekennzeichnet, dass der Schallsensor in Form eines über eine Luftsäule mit einem in der Sonde angeordneten Mikrophon kommunizierenden Ballons ausgebildet ist.

7. Vitaldiagnosegerät nach Anspruch 5, dadurch gekennzeichnet, dass der Schallsensor in Form eines über einen Schlauch mit der Anzeigeeinrichtung verbundenen Ballons ausgebildet ist.

8. Vitaldiagnosegerät nach mindestens einem der vorangehenden Ansprüche, dadurch gekennzeichnet, dass das Gerät weiter mit einem zur Erfassung des pulssynchronen Blutbewegungen dienenden Doppler-Schallkopf versehen ist.

9. Vitaldiagnosegerät nach mindestens einem der vorangehenden Ansprüche, dadurch gekennzeichnet, dass die Anzeigeeinrichtung mit einem die Wiedergabe des EKG erlaubenden Sichtfeld versehen ist.

10. Vitaldiagnosegerät zur Erfassung wenigstens der Herzfunktion mit einer füllerartigen ausgebildeten Sonde, die mit wenigstens zwei voneinander beabstandet am Umfang der Sonde angeordneten Elektroden versehen ist, und einer Einrichtung zum Anzeigen der von der Sonde erfassten, einen Rückschluss auf Vitalfunktionen erlaubenden Signale, gekennzeichnet durch eine flexible, ein Einführen in die Speiseröhre erlaubende Ausgestaltung, wobei die Sonde über ein Kabel mit der gesondert ausgebildeten, außerhalb des Körpers des Patienten verbleibenden Auswerte- und Anzeigeeinrichtung verbunden ist, und die Sonde mit einem Temperatursensor, einem Sensor zur Erfassung der Sauerstoffsättigung, einem zur Erfassung der Herztöne und der Atemgeräusche dienenden Schallsensor und mit einem zur Erfassung der pulssynchronen Blutbewegungen dienenden Doppler-Schallkopf versehen ist, der seine Umgebung segmentweise über einen Raumwinkel von 360° abtastet und dadurch gekennzeichnet, dass in der Auswerte- und Anzeigeeinrichtung dasjenige Raumsegment ausgewertet und angezeigt wird, das das kräftigste pulssynchrone Flüssigkeitsströmungssignal liefert.
